# EUROPEAN PATENT SPECIFICATION

(11) **EP 0 226 827 B1**
(45) Date of publication and mention of the grant of the patent: **03.02.1993**
(21) Application number: 86116075.2
(22) Date of filing: 20.11.1986
(51) Int. Cl.: C07K 1/06, C07K 7/10, C07K 7/00

(54) **Protection of methionine in a polypeptide chain from irreversible oxidation**
Schutz von Methionin in einer Polypeptid-Kette von irreversibler Oxydation
Protection de méthionine dans une chaîne polypeptidique contre une oxydation irréversible

(30) Priority: 18.12.1985 IT 4895585
(43) Date of publication of application: 01.07.1987
(73) Proprietor: ISTITUTO di RICERCA CESARE SERONO SpA, Ardea (Roma) (IT)
(72) Inventor: Canosi, Umberto, I-00041 Albano Laziale (IT); Villa, Stefano, I-00195 Rome (IT)
(74) Representative: Kraus, Walter, Dr.

(56) References cited:
- EP-A- 0 199 018
- CHEMICAL ABSTRACTS, vol. 97, no. 1, 5th July 1982, page 656, no. 6769g, Columbus, Ohio, US; M.BIENERT et al.: "Protection of methionine in peptides during iodination by sulfonium salt formation" & INT. J. PEPT. PROTEIN RES. 1982, 19(3), 310-14
- CHEMICAL ABSTRACTS, vol. 98, no. 13, 28th March 1983, page 643, no. 107746y, Columbus, Ohio, US; M.BODANSZKY et al.: "Experiments on the protection of the thioether in methonine", & INT. J. PEPT. PROTEIN RES. 1982, 20(5), 408-13
- JOURNAL OF THE CHEMICAL SOCIETY, part II, 1951, pages 863-1941; The Chemical Society, London, GB; R.O.ATKINSON et al.: "Sulphonium derivatives of DL-methionine and 5-(2-methylthioethyl)hydantoin"
- JOURNAL OF THE AMERICAN CHEMICAL SOCIETY, vol. 97, no. 13, 25th June 1975, page 3A, ref. no. 3875; W.C.JONES, Jr. et al.: "Specific enrichment with 13C of the methionine methyl groups of sperm whale myoglobin 1"

## Description

Methionine in a polypeptide chain is protected from irreversible oxidation due to oxidizing media by alkylation with an alkyl halogenide and formation of a sulfonium salt resistant to further oxidation.

The present invention relates to the field of peptide chemistry and, more particularly, to a method for protecting a reactive amino acid from irreversible oxidation.

For several years interest has developed in producing by genetically engineered bacterial cells pharmaceuticals of a proteic nature which were formerly prepared by chemical synthesis or extracted from natural sources at relatively high costs.

Such polypeptides, usually large in their size, are often expressed by E.Coli cells directly as mature molecules and differ from the natural molecules due to the presence of a methionine at the amino terminus.

Alternatively, small peptides can be expressed in E.Coli cells as part of hybrid proteins, from which they are separated by chemical or enzymatic reactions specific for a given aminoacid or aminoacid sequence.

Itakura et al. (Science, 198, 1056, 1977) reported the expression of Somatostatin in E.Coli as hybrid protein with beta-galactosidase. Somatostatin is separated from the hybrid by cleavage with cyanogen bromide (CNBr) which splits specifically the methionine residue. Goeddel et al (Proc. Natl. Acad. Sci. USA, 76, 106, 1979) report the expression of the human A and B insulin chains in E.Coli as hybrid proteins with beta-galactosidase.

The pending Italian Patent application No. 47856A/85* reports the expression in E.Coli of the Growth Hormone Releasing Factor (GRF) peptide as a hybrid protein TrpE-GRF.

To obtain the GRF polypeptide, the hybrid protein is subjected to a series of chemical reactions consisting in cleaving the hybrid at the aminoacid tryptophan by treatment with iodosobenzoic acid which separates the GRF segment from the TrpE moiety, the cysteine residue having previously been reduced and carboxyamidomethylated. Unfortunately, treatment with iodosobenzoic acid oxidizes the thioether group of the methionine in position 27 to sulfone. Thus the obtained GRF, although biologically active, differs from native GRF in that its Met27 aminoacid is oxidized to the methionine-sulfone derivative.

The purpose of this invention is to provide a method able to protect methionine from the irreversible oxidation caused by * corresponding to European patent application EP-A-0 199 018 iodosobenzoid acid or other oxidizing agents.

This invention, although described with particular reference to the protection of methionine in position 27 of the GRF peptide, obtained in accordance with the above-mentioned Italian patent application, provides a method which is applicable to any similar situation in which such protection is useful or necessary.

The process of this invention is most suitable when the polypeptide of interest is produced in the form of a fused, hybrid protein in a genetic engineering procedure, as in the above-mentioned examples.

It has been reported that iodoacetic acid and its amide react specifically with the sulphur atom present in methionine. Such reaction has been used to separate methionine-containing peptides from a peptide mixture obtained by tryptic digestion (Degen and Kyte, Anal. Biochem. 89, 529, 1978). It is also known that methyl iodide reacts with the sulphur atom of methionine and this reaction has been used to replace the methyl group of methionine with a radioactive-labeled one (Rothgeb et al, Biochemistry 16, 5813, 1977; Barling et al, Anal. Biochem. 144, 542, 1985).

However, nothing in the prior art suggests use of an alkyl halogenide as a means of protecting methionine.

According to the present invention the protective reaction occurs in accordance with the following scheme (where X = I or Br and R = H, COOH or CONH₂): 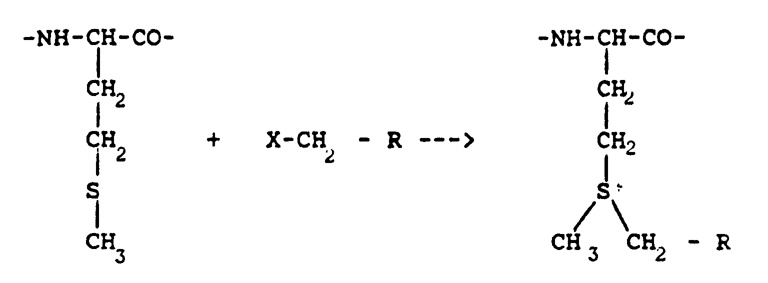
The sulfonium salt thus formed is resistant to further oxidation and, in particular, is resistant to the oxidizing action of iodosobenzoid acid.

Methionine can be regenerated when the sulfonium salt is reacted with a di- or poly-thiole of the general formula: 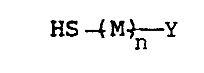
where "n" is an integer comprised between 1 and 4;
M represents a -CH₂- or 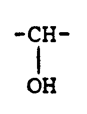
group;
Y represents an -OH or -SH group.

Typical compounds within this general formula are 2-mercaptoethanol and 1,4-dithiothreitol.

The scheme of the regeneration reaction is as follows: 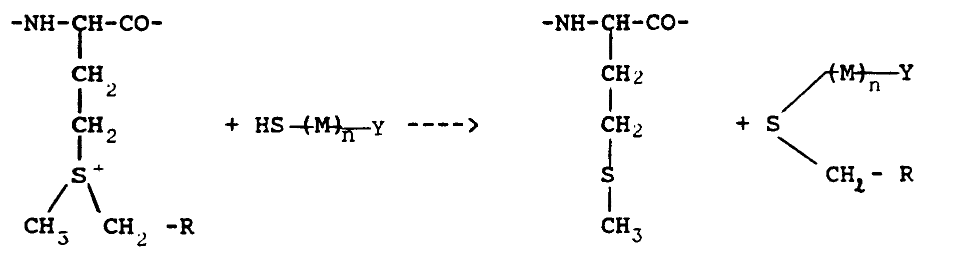
The detailed description which follows refers to the specific example of application of the invention method to the protection of methionine present in position 27 of the GRF peptide, expressed as hybrid protein (TrpE-GRF).

In a specific example iodoacetamide has been used as the alkylating agent. However, it is evident that the invention also encompasses use of other alkyl halogenides able to alkylate the sulphur atom of methionine, such as, for example, bromoacetic acid, iodoacetic acid, their esters and salts, bromoacetamide, alkyl bromides and iodides like methylbromide, ethylbromide and the corresponding iodides, and so on.

The invention also includes the application of the alkylation reaction and the following deprotection reaction to any case where it may be useful or necessary to protect in a reversible way methionine from its irreversible oxidation to methionine sulfone.

### EXAMPLE 1

### Methionine protection

5 mg of hybrid TrpE-GRF44 obtained as described in pending Italian Patent Application No. 47856A/85, previously carboxyamidomethylated, are dissolved in 0.5 ml of the following buffer: 4M Guanidine-HCl dissolved in 30% Acetic acid. To the solution are added 277.5 mg (saturation amount) of iodoacetamide and the mixture is incubated in the dark for 2 hours at 50°C. 1 ml of water is then added and the mixture is allowed to stand for 2 hours at 4°C.

During this incubation period a precipitate is formed and collected by centrifugation for 10 min at 10,000 rpm.

The iodoacetamide reacts with the side group of methionine to form the corresponding S-methyl-carbamino sulfonium salt as shown in the previous scheme.

### Reaction with o-iodosobenzoic acid

5 mg of o-iodosobenzoic acid (IBA) are dissolved in 375 µl of the following buffer: 4M Guanidine-HCl dissolved in 80% acetic acid.

To this solution are added 7.5 µl of p-cresole, and the previously obtained precipitate containing the hybrid protein TrpE-GRF44 protected at methionine 27 is dissolved therein. After 20 hours of incubation in the dark at room temperature, 750 µl of water are added and after 10 min the reaction mixture is centrifuged for 5 mins at 12,000 rpm. In the acqueous phase peptides are present, among them GRF44.

### GRF44 purification and deprotection

The acqueous solution obtained as described above is desalted by gel filtration using a Sephadex G25, 1x25 cm column, equilibrated with 5% acetic acid. The flow rate is about 12 ml/hr. The excluded material is rapidly dessicated by means of evaporation and recovered in 500 µl of Hepes 0.2M pH8,9 containing 0.12M mercaptoethanol. The resulting mixture is incubated at 37°C for 24 hrs and then acidified with Trifluoroacetic acid (pH=4).

The GRF44 is subsequently purified by HPLC, using a C18 column equilibrated with 0.1% Trifluoroacetic acid and eluted with 0.05% Trifluoroacetic acid in acetonitrile. Study of the aminoacid sequence shows that the residues of the obtained GRF 44 are identical to those of natural GRF44.

### EXAMPLE 2

### Protection of methionine

5 mg of hybrid TrpE-GRF44 protein obtained as described in the Italian Patent Application No. 47856A/85, previously carboxyamidomethylated, are dissolved in 0.5 ml of the following buffer: 4M Guanidine-HCl dissolved in 30% acetic acid.

To the solution are added 90 µl of methyliodide and the mixture is shaken in the dark for 18 hours at 37°C. 1 ml of water is then added and the mixture is allowed to stand for 2 hours at 4°C.

During this incubation period a precipitate is formed and collected by centrifugation for 10 min at 10,000 rpm.

The methyliodide reacts with the side group of methionine to form the corresponding S-methyl sulfonium salt as shown in the previous scheme.

### Reaction with o-iodosobenzoic acid

5 mg of o-iodosobenzoic acid (IBA) are dissolved in 375 µl of the following buffer: 4M Guanidine-HCl dissolved in 80% acetic acid.

To this solution are added 7.5 µl of p-cresole and the previously obtained precipitate containing the hybrid TrpE-GRF44 protected at methionine 27 is dissolved therein. After 20 hours of incubation in the dark at room temperature, 750 µl of water are added and after 10 mins the reaction mixture is centrifuged for 5 mins at 12,000 rpm. In the acqueous phase peptides are present, among them GRF44.

### GRF44 purification and deprotection

The acqueous solution previously obtained is desalted by gel filtration. A Sephadex G25, 1x25 cm column, equilibrated with 5% acetic acid is used. The flow rate is about 12 ml/hr. The excluded material is rapidly dessicated by evaporation and recovered in 500 µl of 0.2M N-ethylmorpholine pH9 containing 0.5M Dithiothreitol. The deprotection reaction mixture is incubated at 37° C for 24 hours and then acidified with Trifluoroacetic acid (pH=4).

The GRF44 is subsequently purified by HPLC using a C18 column equilibrated with 0.1% Trifluoroacetic acid and eluted with 0.05% Trifluoroacetic acid in acetonitrile. Study of the aminoacid sequence shows that the GRF 44 obtained by the method described in this invention has residues identical to those of natural GRF44.

## Claims

1. A method of separating a methionine containing polypeptide from a hybrid protein which contains it while protecting said methione from irreversible oxidation which comprises:
reacting said hybrid protein with an alkyl halogenide to form a hybrid protein containing a methionine sulfonium salt containing polypeptide;
cleaving the methionine sulfonium salt containing polypeptide from the hybrid protein; and
reacting the methionine sulfonium salt containing polypeptide with a mono- or di-thiol to recover the methionine containing polypeptide.

2. The method of claim 1 wherein the alkyl halogenide has the formula X-CH₂-R wherein X is I or Br and R is H, CH₃, COOH or CONH₂.

3. The method of claim 2 wherein the mono- or di-thiol has the formula HS-(M)ₙ-Y wherein n is an integer of 1 to 4, M is -CH₂- or -CH(OH)-, and Y is -OH or SH.

4. The method of claim 3 wherein the methionine containing polypeptide is GRF.

5. The method of claim 4 wherein the hybrid protein is TrpE-GRF.

6. The method of claim 5 wherein the GRF is cleaved from the hybrid protein by reaction with iodosobenzoic acid.

7. The method of claim 6 wherein the alkyl halogenide is iodoacetamide or methyl iodide.

8. The method of claim 5 wherein the mono- or di-thiol is mercaptoethanol or 1,4-dithiothreitol.

## Patentansprüche

1. Verfahren zur Abtrennung von einem Methionin-enthaltenden Polypeptid aus einem Hybrid-Protein, das es enthält, wobei das Methionin von der irreversiblen Oxidation geschützt ist, dadurch **gekennzeichnet,** daß
das Hybrid-Protein mit einem Alkylhalogenid unter Bildung eines Hybrid-Proteins, das ein Methionin-Sulfoniumsalzenthaltendes Polypeptid enthält, umgesetzt wird;
das Methionin-Sulfoniumsalz-enthaltende Polypeptid von dem Hybrid-Protein abgespalten wird; und
das Methionin-Sulfiniumsalz-enthaltende Polypeptid mit einem Mono- oder Dithiol zur Gewinnung des Methionin-enthaltenden Polypeptids umgesetzt wird.

2. Verfahren nach Anspruch 1, dadurch **gekennzeichnet,** daß das Alkylhalogenid die Formel X-CH₂-R aufweist, worin X I oder Br und R H, CH₃, COOH oder CONH₂ bedeuten.

3. Verfahren nach Anspruch 2, dadurch **gekennzeichnet,** daß das Mono- oder Dithiol die Formel HS-(M)ₙ-Y besitzt, worin n eine ganze Zahl von 1 bis 4, M -CH₂- oder -CH(OH)- und Y -OH oder SH bedeuten.

4. Verfahren nach Anspruch 3, dadurch **gekennzeichnet,** daß das Methionin-enthaltene Polypeptid GRF ist.

5. Verfahren nach Anspruch 4, dadurch **gekennzeichnet,** daß das Hybrid-Protein TrpE-GRF ist.

6. Verfahren nach Anspruch 5, dadurch **gekennzeichnet,** daß das GRF von dem Hybrid-Protein durch Umsetzung mit Iodosobenzoesäure abgespalten wird.

7. Verfahren nach Anspruch 6, dadurch **gekennzeichnet,** daß das Alkylhalogenid Iodacetamid oder Methyliodid ist.

8. Verfahren nach Anspruch 5, dadurch **gekennzeichnet,** daß das Mono- oder Dithiol Mercaptoethanol oder 1,4-Dithiothreit ist.

## Revendications

1. Procédé de séparation d'un polypeptide contenant de la méthionine et d'une protéine hybride qui contient celui-ci, tout en protégeant ladite méthionine contre une oxydation irréversible, comprenant les étapes consistant à:
- faire réagir ladite protéine hybride avec un halogénure d'alcoyle pour former une protéine hybride contenant un polypeptide contenant le sel de sulfonium de méthionine ;
- séparer le polypeptide contenant le sel de sulfonium de méthionine de la protéine hybride ; et
- faire réagir le polypeptide contenant le sel de sulfonium de méthionine avec un mono- ou dithiol pour récupérer le polypeptide contenant la méthionine.

2. Procédé selon la revendication 1, dans lequel l'halogénure d'alcoyle répond à la formule X-CH₂-R où X est I ou Br, et R est H, CH₃, COOH ou CONH₂.

3. Procédé selon la revendication 2, dans lequel le mono- ou dithiol répond à la formule HS-(M)ₙ-Y où n est un nombre entier valant de 1 à 4, M est -CH₂- ou -CH(OH)-, et Y est -OH ou SH.

4. Procédé selon la revendication 3, dans lequel le polypeptide contenant la méthionine est GRF.

5. Procédé selon la revendication 4, dans lequel la protéine hybride est TrpE-GRF.

6. Procédé selon la revendication 5, dans lequel le GRF est séparé de la protéine hybride par réaction avec l'acide iodosobenzoïque.

7. Procédé selon la revendication 6, dans lequel l'halogénure d'alcoyle est l'iodoacétamide ou l'iodure de méthyle.

8. Procédé selon la revendication 5, dans lequel le mono- ou dithiol est le mercaptoéthanol ou le 1,4-dithiothreitol.
